# DEMANDE DE BREVET EUROPEEN

(11) **EP 2 371 970 A2**
(43) Date de publication de la demande: **05.10.2011**
(21) Numéro de dépôt: 10186055.9
(22) Date de dépôt: 25.03.2002
(51) Int. Cl.: C12Q 1/68, C12N 15/10

(54) **Procédés de purification et de détection de séquences cibles d'ADN double brin par intéraction triple hélice**

(30) Priorité: 23.03.2001 FR 0103953; 23.04.2001 US 285272 P
(62) Demande divisionnaire de: 02722374.2
(71) Demandeur: Aventis Pharma S.A., 92160 Antony (FR)
(72) Inventeur: Blanche, Francis, 75013, PARIS (FR); Cameron, Béatrice, 75013, PARIS (FR)
(74) Mandataire: Bouvet, Philippe

(57) **Abrégé**

La présente invention a pour objet de nouvelles séquences cibles d'ADN double brin susceptibles d'interagir avec un troisième brin et de former une triple hélice stable. La présente invention concerne en outre un procédé de purification d'une molécule d'ADN double brin selon lequel on met en contact une solution contenant ladite molécule d'ADN avec un troisième brin d'ADN capable de former par hybridation une structure triple hélice avec une séquence cible d'ADN double brin portée par ladite molécule d'ADN.

## Description

La présente invention a pour objet de nouvelles séquences d'ADN cibles capables de former des structures de type triple hélice ainsi qu'un nouveau procédé pour la purification d'ADN. Plus particulièrement, le procédé de purification selon l'invention met en oeuvre une hybridation entre une séquence d'ADN cible et un oligonucléotide. Le procédé selon l'invention s'avère être particulièrement utile puisqu'il permet de purifier de l'ADN double brin de qualité pharmaceutique avec des rendements élevés.

La présente invention a également pour objet de nouvelles méthodes de détection, de quantification, d'isolement ou de tri de molécules d'ADN contenant lesdites séquences spécifiques cibles.

Les procédés de purification selon l'invention reposent essentiellement sur une interaction triple hélice entre une séquence d'ADN cible particulière et un oligonucléotide composé de bases naturelles ou modifiées.

Il a été montré que des oligonucléotides homopyrimidiques sont capables d'interagir spécifiquement dans le grand sillon de la double hélice d'ADN pour former localement des structures à trois brins appelées triple hélices (Moser et al., Science 238 (1987) 645 ; Povsiz et al., J. Am. Chem 111 (1989) 3059). Ces oligonucléotides reconnaissent sélectivement la double hélice d'ADN au niveau de séquences oligopurine-oligopyrimidine, c'est à dire au niveau de régions possédant une séquence oligopurique sur un brin et une séquence oligopyrimidique sur le brin complémentaire, et y forment localement une triple hélice. Les bases du troisième brin oligonucléotidique homopyrimidique forment des liaisons hydrogène (liaisons de type Hoogsteen) avec les purines des paires de bases Watson-Crick.

De la même façon, des structures de type triple hélice peuvent se former entre un oligonucléotide homopurique et un ADN double brin homopurique-homopyrimidique. Dans ce type de structure les bases puriques de l'oligonucléotide forment des liaisons de type Hoogsteen inverse avec les bases puriques de l'ADN double brin.

Ces interactions triple hélice site-spécifiques ont été notamment mises en oeuvre par Looney et al. (Science 241 (1988) 456) pour le contrôle de l'expression de certains gènes, et par Hélène et al. (BBA 1049 (1990) 99 ; WO95/18223) qui décrivent la formation de structures triple hélice entre des oligonucléotides et des séquences cibles présentes au sein de promoteurs ou de régions codantes et ainsi la possibilité de moduler le profil d'expression de ces gènes, vraisemblablement par une action inhibitrice de l'ARN polymérase au niveau de l'initiation et/ou de l'élongation.

Ce type d'interaction triple hélice pour la purification d'ADN plasmidique à partir d'un mélange complexe qui contient la molécule d'ADN en mélange avec d'autres composants, a été également décrit dans la demande internationale WO96/18744 pour la purification d'ADN plasmidique. Plus particulièrement, cette demande décrit un procédé de purification d'ADN double brin consistant à mettre en contact le mélange complexe et un support sur lequel se trouve couplé de façon covalente un oligonucléotide capable de former par hybridation une triple hélice avec une séquence spécifique de l'ADN cible.

Dans cette interaction spécifique de type triple hélice à des fins de purification, la spécificité est due à des appariements mettant en jeu des liaisons hydrogène de type Hoogsteen entre des bases thymine (T) du troisième brin constitué par l'oligonucléotide d'une part et des paires de bases AT de l'ADN double brin d'autre part, pour former des triades T*AT. De même, des cytosines protonées situées dans le troisième brin s'apparient à des paires de base GC de l'ADN double brin pour former des triades ⁺C*GC (Sun et al., Curr. Opin Struc Biol. 3 (1993) 345). Il a été jusqu'à présent établi que ces triades T*AT et ⁺C*GC (appelées triades canoniques) assurent une stabilité maximale de la triple hélice. Cependant, de nombreux autres facteurs interviennent également dans la stabilisation de la triple hélice, tels que par exemple le pourcentage de cytosines, le pH, la salinité du milieu ou l'environnement de la triple hélice. Il est également largement décrit que l'introduction de triades dites non canoniques (c'est à dire différentes des triades T*AT et ⁺C*GC) provoque une déformation structurale plus ou moins importante au niveau de la triple hélice, et entraîne systématiquement une déstabilisation significative de celle-ci. L'introduction de différentes triades non canoniques a été d'ailleurs étudiée dans le cadre d'études comparatives (Roberts et al., Proc. Natl. Acad. Sci. USA 88, 9397 ; Fossella et al., (1993) Nucleic Acids Research 21, 4511 ; Govers et al., Nucleic Acids Research (1997) 25, 3787) montrant une déstabilisation variable de la triple hélice en fonction de la nature de la triade non canonique introduite.

Si ce procédé permet une purification rapide et efficace d'un ADN cible de qualité pharmaceutique, il nécessite toutefois qu'une séquence suffisamment longue, de préférence homopurique parfaite, soit présente sur un des deux brins de l'ADN à purifier, et qu'elle soit complémentaire au troisième brin d'ADN. Cette séquence peut être naturellement présente ou être insérée artificiellement au sein de la séquence cible d'ADN double brin que l'on souhaite purifier.

Le demandeur a maintenant découvert de manière surprenante et inattendue qu'une molécule d'ADN portant sur un brin une séquence d'ADN cible non essentiellement composée de bases puriques était également susceptible de former une structure triple hélice stable avec un troisième brin d'ADN, malgré la présence de bases non complémentaires à celles de l'oligonucléotide conduisant à la formation de triades non canoniques.

Plus précisément, les séquences d'ADN double brin cibles nouvellement identifiées par le demandeur comprennent sur un brin une séquence homopurique interrompue par un nombre déterminé de bases pyrimidiques. Le demandeur a également découvert que ces séquences d'ADN homopuriques-homopyrimidiques imparfaites pouvaient être utilisées pour purifier efficacement les molécules d'ADN les contenant par interaction triple hélice.

Les séquences nouvellement identifiées sont en outre particulièrement utiles pour la détection, la quantification, l'isolement ou le tri de molécules d'ADN les contenant.

La présente invention a donc pour objet de nouvelles séquences d'ADN cible comprenant sur un brin une séquence ayant la formule générale suivante :

5' -(R)ₙ-(N)ₜ-(R')ₘ-3'

dans laquelle :
R et R' représentent des nucléotides composés uniquement de bases puriques ;
n et m sont des nombres entiers inférieurs à 9, et la somme n+m est supérieure à 5 ;
N est une séquence nucléotidique comprenant à la fois des bases puriques et des bases pyrimidiques ;
t est un nombre entier inférieur à 8 ;
ladite séquence d'ADN étant susceptible d'interagir avec un troisième brin d'ADN et ainsi de conduire à la formation d'une structure triple hélice.

Les séquences homopuriques R et R' situées respectivement dans les parties 5' et 3' de la séquence d'ADN cible, ont donc une longueur totale supérieure ou égale à 6. Elles comprennent les bases adénine et guanine susceptibles d'interagir avec un troisième brin afin de conduire à la formation d'une structure triple hélice constituée de triades canoniques T*AT et ⁺C*GC. De préférence, les séquences homopuriques R et R' comprennent au moins 2 guanines au total et au moins 2 adénines. De manière encore plus préférentielle, ces séquences puriques comprennent un motif du type (AAG).

La séquence centrale N a une longueur t inférieure à 8 paires de bases puriques et pyrimidiques et est capable selon l'invention d'interagir avec un troisième brin d'ADN, afin de conduire à la formation de triades non canoniques. De préférence, la séquence centrale N a une longueur supérieure ou égale à 1 et inférieure à 8. De manière encore plus préférentielle, la séquence centrale N a une longueur supérieure ou égale à 2 et inférieure à 8.

On entend par triade canonique, les deux triades nucléotidiques résultant de l'interaction des doublet AT et GC de l'ADN double brin avec les bases T et ⁺C pour donner respectivement les triades T*AT et ⁺C*GC. Ces deux triades sont parmi les 16 triades existantes, celles ayant la plus forte stabilité.

On entend par triade non canonique, l'ensemble des 14 autres triades nucléotidiques. Elles résultent de l'interaction d'un ADN double brin avec un troisième brin d'ADN de manière non spécifique et présentent une stabilité moindre par rapport aux triades canoniques T*AT et ⁺C*GC. On peut citer en particulier les triades non canoniques T*CG et T*GC qui sont formées respectivement par interaction entre un doublet CG ou GC de la séquence cible et une thymine (T) du troisième brin, la triade non canonique G*CG résultant de l'interaction entre un doublet CG de la séquence cible et une guanine (G) du troisième brin, les triades non canoniques C*AT et C*TA qui résultent de l'interaction respectivement des doublets AT et TA de la séquence cible et de cytosines (C) situées sur le troisième brin, la triade non canonique G*CG qui est formée par interaction d'un doublet CG avec une guanine (G) du troisième brin, ou encore la triade T*TA qui résulte de l'interaction d'un doublet TA avec une thymine (T) du troisième brin.

Il est bien entendu que, comme les extrémités puriques en 5' et en 3', la séquence centrale N peut aussi former des triades canoniques T*AT et ⁺C*GC résultant de l'interaction respective de doublets AT et GC avec des bases thymine (T) et cytosine (C) situées sur le troisième brin D'ADN.

De préférence, la séquence centrale N comprend des bases puriques et pyrimidiques conduisant à la formation d'au plus 6 triades non canoniques. De manière plus préférentielle, les triades non canoniques résultant de l'interaction de la partie centrale avec l'oligonucléotide sont choisies parmi les triades non canoniques T*CG, T*GC, C*AT, et C*TA. A titre d'exemples de répartitions préférées de ces triades, on peut citer la formation de six triades non canoniques comprenant une C*AT, une C*TA, deux T*CG, et deux T*GC, la formation de cinq triades non canoniques comprenant deux C*AT et trois T*GC, ou encore la formation de trois triades non canoniques comprenant deux T*GC et une C*AT. Plusieurs triades non canoniques T*TA peuvent également être présentes, mais dans ce cas, elle ne sont pas placées consécutivement au sein de la triple hélice.

La séquence centrale comprend de préférence au plus trois bases pyrimidiques C ou T conduisant à la formation des triades non canoniques T*CG et C*TA ou G*TA. Préférentiellement, les trois bases pyrimidiques ne sont pas consécutives mais sont espacées par des bases puriques A ou G, qui peuvent interagir avec le troisième brin d'ADN pour former les bases non canoniques T*GC et C*AT ainsi que des triades canoniques T*AT et ⁺C*GC.

Selon un mode de réalisation particulier de l'invention, la séquence cible d'ADN double brin est la séquence 5'-AA GAA GCA TGC AGA GAA GAA-3' (SEQ ID N°: 1).

Le troisième brin d'ADN qui est susceptible d'interagir avec les séquences d'ADN double brin selon l'invention peut-être par exemple un oligonucléotide ou le brin d'un autre ADN double brin à l'état désapparié localement, et peut contenir les bases suivantes :
- la thymine (T), qui est capable de former des triades canoniques T*AT avec les doublets AT de la séquence cible d'ADN double brin, ainsi que des triades non canoniques T*CG et T*GC respectivement avec les doublets CG et GC de la séquence d'ADN cible (Soyfer et al., in Triple Helical Nucleic Acids (1996) Springer, New York, pp. 151-193) ;
- la guanine (G) qui est capable de former des triades G*TA avec les doublets TA de l'ADN double brin (Soyfer et al., in Triple Helical Nucleic Acids (1996) Springer, New York, pp. 151-193) ;
- la cytosine (C) qui est capable de former des triades canoniques ⁺C*GC (cytosine protonée C⁺) ou non canoniques C*AT et C*TA, respectivement avec les doublets GC, AT, et TA de l'ADN double brin cible ; et
- l'uracile qui est capable de former des triplets avec les paires de base AU ou AT de la séquence cible (Bates et al., Nucleic Acids Research 23 (1995) 3627).

De préférence, le troisième brin d'ADN utilisé comprend une séquence homopyrimidique riche en cytosines, qui sont présentes à l'état protoné en pH acide et stabilisent la triple hélice. De tels oligonucléotides peuvent comprendre par exemple la séquence (CCT)n, la séquence (CT)n ou la séquence (CTT)n, dans laquelle n est un nombre entier compris entre 1 et 20 inclus. Il est particulièrement avantageux d'utiliser des séquences de type (CT)n, (CTT)n, ou bien des séquences où sont combinés des motifs (CCT), (CT) ou (CTT).

Lorsque le troisième brin d'ADN se présente sous la forme d'un oligonucléotide, celui-ci peut être naturel, c'est à dire composé de bases naturelles, non modifiées, ou encore modifié chimiquement. En particulier, l'oligonucléotide peut présenter avantageusement certaines modifications chimiques permettant d'augmenter sa résistance ou sa protection vis à vis des nucléases, ou son affinité vis à vis de la séquence spécifique.

Selon la présente invention, on entend par oligonucléotide tout enchaînement de nucléosides ayant subi une modification du squelette dans le but de le rendre plus résistant aux nucléases. Parmi les modifications possibles on peut citer, les oligonucléotides phosphorothioates qui sont capables de former des triples hélices avec l'ADN (Xodo et al., Nucleic Acids Research, 22 (1994) 3322), de même que les oligonucléotides possédant des squelettes formacétal ou méthylphosphonate (Matteucci et al., J. Am. Chem. Soc., 113 (1991) 7767). On peut également utiliser les oligonucléotides synthétisés avec des α-anomères de nucléotides, qui forment également des triples hélices avec l'ADN (Le Doan et al., Nucleic Acids Research, 15 (1987) 7749). Une autre modification du squelette est la liaison phosphoramidate. On peut citer par exemple la liaison internucléotidique N3'-P5' phosphoramidate décrite par Gryaznov et al. (J. Am. Chem. Soc., 116 (1994) 3143), qui donne des oligonucléotides formant avec l'ADN des triple hélices particulièrement stables. Parmi les autres modifications du squelette, on peut citer également l'utilisation de ribonucléotides, de 2'-O-méthylribose, ou de phosphodiester (Sun et al., Curr. Opinion in Struct. Biol., 3 (1993) 3143). Le squelette phosphoré peut enfin être remplacé par un squelette polyamide comme dans les PNA (Peptide Nucleic Acid), qui peuvent également former des triple hélices (Nielsen et al., Science, 254 (1991) 1497 ; Kim et al., J. Am. Chem. Soc., 115 (1993) 6477-6481).

La thymine du troisième brin peut aussi être remplacée par un 5-bromouracile, ce qui augmente l'affinité de l'oligonucléotide pour l'ADN (Povsic et al., J. Am. Chem. Soc., 111 (1989) 3059). Le troisième brin peut également contenir des bases non naturelles, parmi lesquelles on peut citer la 7-déaza-2'-déoxyxanthosine (Milligan et al., Nucleic Acids Res., 21 (1993) 327), la 1-(2-déoxy-alpha-D-ribofuranosyl)-3-méthyl-5-amino-1*H*-pyrazolo[4,3-*d*]pyrimidine-7-one (Koh et al., J. Am. Chem. Soc., 114 (1992) 1470), la 8-oxoadénine, la 2-aminopurine, la 2'-O-méthyl-pseudoisocytidine, ou toute autre modification connue de l'homme du métier (Sun et al., Curr. Opinion in Struct. Biol., 3 (1993) 345).

Un autre type de modification du troisième brin a plus particulièrement pour objet d'améliorer l'interaction et/ou l'affinité entre le troisième brin et la séquence spécifique. En particulier, une modification tout à fait avantageuse selon l'invention consiste à méthyler les cytosines de l'oligonucléotide en position 5. L'oligonucléotide ainsi méthylé présente la propriété remarquable de former une triple hélice stable avec la séquence spécifique dans des zones de pH plus proches de la neutralité (≥ 5 ; Xodo et al., Nucleic Acids Research 19 (1991) 5625). Il permet donc de travailler à des pH plus élevés que les oligonucléotides de l'art antérieur, c'est à dire à des pH où les risques de dégradation de l'ADN plasmidique sont bien inférieurs.

La longueur peut être adaptée au cas par cas par l'homme du métier en fonction de la sélectivité et de la stabilité de l'interaction recherchées.

Les troisièmes brins d'ADN selon l'invention peuvent être synthétisés par toute technique connue. En particulier, ils peuvent être préparés au moyen de synthétiseurs d'acides nucléiques. Toute autre méthode connue de l'homme du métier peut bien évidemment être utilisée.

Ces troisièmes brins d'ADN ou ces oligonucléotides sont capables de former une triple hélice avec une séquence spécifique d'ADN double brin telle que précédemment décrite, comportant une région interne N mixte (pyrimidique-purique) d'une longueur inférieure à 8 nucléotides flanquée par deux régions homopuriques R et R'. Ces dernières peuvent par exemple comprendre un motif du type GAA.

A titre d'exemple, on peut citer la séquence d'ADN double brin cible correspondant à la séquence : 5'- AA GAA GCA TGC AGA GAA GAA -3' (SEQ ID N° : 1), qui est capable de former une triple hélice avec un oligonucléotide comprenant une séquence choisie parmi les séquences suivantes:
5'- TT CTT CTT CTT CTT CTT CTT - 3' (SEQ ID N° : 2),
5' ― TT CTT CTT GCT TCT CTT CTT ― 3' (SEQ ID N°: 3),
5' ― TT CTT CTT GTT TCT CTT CTT ― 3' (SEQ ID N°: 4), et
5' ― TT CTT CTT CCT TCT CTT CTT ― 3' (SEQ ID N°: 5).

La formation de la triple hélice pourra être obtenue en présence d'ions Mg²⁺ qui peuvent éventuellement favoriser la stabilisation de cette structure (Vasquez et al., Biochemistry 34 (1995) 7243 ; Beal et al., Science 251 (1991) 1360).

Selon un mode de réalisation préféré, les séquences d'ADN cibles selon l'invention peuvent être présentes naturellement sur l'ADN double brin et il est alors particulièrement intéressant d'utiliser un oligonucléotide capable de former une triple hélice avec une telle séquence présente par exemple dans la séquence de gènes d'intérêt tels que des gènes d'intérêt thérapeutique ou expérimental, ou des gènes marqueurs. A cet égard, la demanderesse a analysé les séquences nucléotidiques de différents gènes d'intérêt et à testé la stabilité des interactions triple hélice avec un oligonucléotide du type (CTT)n, et elle a pu montrer que certaines régions de ces gènes conduisaient à la formation d'une triple hélice stable malgré la présence de triades non canoniques telles que T*CG, T*GC, C*AT, C*TA et T*TA.

Parmi les séquences qui sont naturellement présentes sur un ADN double brin, on peut citer la séquence 5'- AA GAA GCA TGC AGA GAA GAA ― 3' (désignée ID1) (SEQ ID N°:1) présente dans la séquence du gène humain FGF1 (Jaye et al., Science 233 (1986) 541), la séquence 5' - GAA GAA GCA CGA GAA G - 3' (SEQ ID N° :6) du gène humain codant pour le facteur IX impliqué dans la coagulation (Kurachi et al., Proc. Natl. Acad. Sci. U.S.A. 79 (1982) 6461), les séquences 5' - AAA GAA AGC AGG GAA G - 3' (SEQ ID N° : 7) et 5' - GAA GAG GAA GAA G - 3' (SEQ ID N°: 8) du gène de la phosphatase alcaline sécrétée SeAP (Millan et al., J. Biol. Chem., 261 (1986) 3112), la séquence 5' - AAG GAG AAG AAG AA - 3' (SEQ ID N° :9) du gène humain de l'alpha foeto-protéine hαFP (Gibbs et al., Biochemistry 26 (1987) 1332), et la séquence 5' - AA GAT GAG GAA GAA G - 3' (SEQ ID N° :10) du gène humain GAX permettant de contrôler la resténose (Gorski et al., Mol. Cell. Biol., 13 (1993) 3722) enfin la séquence 5'- GGC AAC GGA GGA A-3' (SEQ ID N :13) du gène VEGFB-167 humain (Olofsson et al., J. Biol. Chem., 271 (1996) 19310). La formation d'une triple hélice avec une séquence présente dans un gène d'intérêt thérapeutique ou expérimental est particulièrement avantageuse dans la mesure où la séquence cible est naturellement présente sur la molécule d'ADN double brin et il n'est pas nécessaire de modifier l'ADN double brin cible ou le plasmide portant ce gène pour lui incorporer une séquence spécifique artificielle. Alternativement une séquence cible peut également être introduite de manière artificielle au sein de l'ADN double brin.

Un deuxième aspect de la présente invention réside dans un procédé pour la purification d'ADN double brin, selon lequel on met en contact une solution contenant un ADN, en mélange avec d'autres composants, avec un troisième brin d'ADN tel que précédemment décrit, qui est alors de préférence un oligonucléotide capable de former par hybridation une triple hélice avec une séquence spécifique présente sur l'ADN double brin telle que décrite ci-dessus. De préférence, l'ADN double brin est mis en contact en solution avec l'oligonucléotide immobilisé sur un support. De manière encore plus préférentielle, l'oligonucléotide est couplé de façon stable, covalente ou non covalente au dit support. Ainsi, l'étape de mise en contact d'une solution contenant un ADN double brin peut avantageusement consister à faire passer la solution d'ADN, en mélange avec d'autres composants, sur le support auquel est couplé de l'oligonucléotide, afin d'obtenir l'ADN double brin que l'on souhaite purifier de manière efficace et rapide.

De tels supports sont bien connus de l'homme du métier et comprennent par exemple constitué de billes ou de microparticules telles que les particules de latex, ou de tout autre support en suspension. L'oligonucléotide peut être également greffé sur une molécule de type polymère d'origine naturelle ou synthétique. De préférence, le polymère sur lequel est fixé l'oligonucléotide présente une propriété lui permettant d'être facilement séparé de la solution après formation de la triple hélice avec l'ADN double brin. Parmi les polymères naturels, on peut citer les protéines, des lipides, des sucres, ou des polyols. Parmi les polymères synthétiques, on peut citer les polyacrylamides, les polyéthylène glycol, les dérivés styréniques ou les polymères thermosensibles comme par exemple les composés de type poly(N-isopropylacrylamide), qui sont solubles à basse température et deviennent insolubles au dessus de leur température de transition de phase (T. Mori et al., Biotechnology and Bioengineering, 72 (2001) 261).

Le procédé de purification selon la présente invention est particulièrement utile puisqu'il permet i) la purification de molécules d'ADN ne contenant pas de séquence homopurique de longueur suffisamment grande pour permettre la formation d'une structure triple hélice stable avec un oligonucléotide homopyrimidique, mais également ii) les molécules d'ADN dont la séquence homopurique est interrompue par plusieurs bases pyrimidiques. En plus de permettre la purification d'une plus grande variété de molécules d'ADN, ce procédé est également rapide et conduit à des rendements et à des degrés de pureté particulièrement élevés.

Par ailleurs, il permet de purifier des molécules d'ADN à partir de mélanges complexes comprenant d'autres acides nucléiques, des protéines, des endotoxines (telles que les lipopolysaccharides), des nucléases, etc..., et d'obtenir un ADN purifié de qualité pharmaceutique.

Pour permettre son couplage covalent sur le support, l'oligonucléotide est généralement fonctionnalisé. Ainsi, il peut être modifié par un groupement terminal thiol, amine ou carboxyle, en position 5' ou 3'. En particulier, l'ajout d'un groupe thiol, amine ou carboxyle permet, par exemple, de coupler l'oligonucléotide sur un support portant des fonctions disulfure, maléimide, amine, carboxyle, ester, époxyde, bromure de cyanogène ou aldéhyde. Ces couplages se forment par établissement de liaisons disulfure, thioether, ester, amide ou amine entre l'oligonucléotide et le support. Toute autre méthode connue de l'homme du métier peut être utilisée, telle que des réactifs de couplage bifonctionnels, par exemple.

Par ailleurs, pour améliorer l'hybridation avec l'oligonucléotide couplé, il peut être avantageux que l'oligonucléotide contienne un " bras " et une séquence de bases " espaceur ". L'utilisation d'un bras permet en effet de fixer l'oligonucléotide à une distance choisie du support permettant d'améliorer les conditions d'interaction avec l'ADN. Le bras est avantageusement constitué d'une chaîne carbonée linéaire, comprenant 1 à 18, et de préférence 6 à 12 groupes de type CH₂, et d'une amine qui permet la liaison à la colonne. Le bras est relié à un phosphate de l'oligonucléotide ou d'un " espaceur " composé de bases n'interférant pas avec l'hybridation. Ainsi, " l'espaceur " peut comprendre des bases puriques. A titre d'exemple, " l'espaceur " peut comprendre la séquence GAGG.

L'oligonucléotide couplé au support de purification peut avoir par exemple la séquence 5'- GAGG CTT CTT CTT CTT CTT CTT CTT - 3' (GAGG (CTT)₇; SEQ ID N° : 11) dans lequel les bases GAGG ne sont pas impliquées dans une structure triple hélice mais permettent de former un espace entre l'oligonucléotide et le bras de couplage.

Pour la mise en oeuvre de la présente invention, différents types de supports peuvent être utilisés. Il peut s'agir de supports de chromatographie fonctionnalisés, en vrac ou préconditionnés en colonne, de surfaces plastiques fonctionnalisées ou de billes de latex fonctionnalisées, magnétiques ou non. Il s'agit préférentiellement de supports de chromatographie pour la perméation de gel. A titre d'exemple, les supports de chromatographie pouvant être utilisés sont I' agarose, l'acrylamide ou les dextrans ainsi que leurs dérivés (tels que Sephadex®, Sepharose®, Superose®,...), les polymères tels que le poly(styrènedivinylbenzène), ou la silice greffée ou non greffée, par exemple. Les colonnes de chromatographie peuvent fonctionner en mode de diffusion ou de perfusion, ou encore dans un système dit à " lit fluidisé " ou " expansé " en utilisant un support chromatographique dont la densité est adaptée à ce mode particulier de mise en oeuvre.

Le procédé selon la présente invention peut être utilisé pour purifier tout type d'ADN double brin. Il s'agit par exemple d'ADN circulaire, tel qu'un minicercle (Darquet et al., Gene Therapy 6 (1999) 209), un fragment linéaire, un plasmide portant généralement un ou plusieurs gènes d'intérêt thérapeutique ou expérimental. Ce plasmide peut porter également une origine de réplication par exemple de type conditionnelle (tels que les plasmides pCOR qui sont décrits par Soubrier et al., Gene Therapy 6 (1999) 1482), un gène marqueur, etc. Le procédé de l'invention peut être appliqué directement à un lysat cellulaire. Dans ce mode de réalisation, le plasmide, amplifié par transformation puis culture cellulaire, est purifié directement après lyse des cellules. Le procédé selon l'invention peut également être appliqué à un lysat clair, c'est à dire au surnageant obtenu après neutralisation et centrifugation du lysat cellulaire. Il peut bien évidement être appliqué également à une solution pré-purifiée par des méthodes connues. Ce procédé permet aussi de purifier de l'ADN, linéaire ou circulaire, portant une séquence d'intérêt, à partir d'un mélange comprenant des ADN de différentes séquences. Le procédé selon l'invention peut également être utilisé pour la purification d'ARN double brin.

Le lysat cellulaire peut être un lysat de cellules procaryotes ou eucaryotes. S'agissant de cellules procaryotes, on peut citer par exemple les bactéries E. *coli, B. subtilis, S. typhimurium, S. aureus,* ou *Streptomyces.* S'agissant de cellules eucaryotes, on peut citer les cellules animales, les levures, les champignons, etc..., et plus particulièrement, les levures *Kluyveromyces* ou *Saccharomyces* ou les cellules COS, CHO, Cl27, NIH3T3, MRC5, 293, etc....

Le procédé de l'invention est particulièrement avantageux puisqu'il permet d'obtenir, de manière rapide et simple, de l'ADN plasmidique de très haute pureté. En particulier, comme illustré dans les exemples, ce procédé permet de séparer efficacement l'ADN plasmidique de composants contaminants, tels que l'ADN chromosomique fragmenté, les ARN, les endotoxines, les protéines, ou les nucléases.

Le procédé de l'invention est en outre utile pour la purification et l'enrichissement de molécules d'ADN, et particulièrement de gènes d'intérêt thérapeutique tels que le gène FGF1, qui sont produits et purifiés à une échelle industrielle, et dont la pureté doit être compatible avec un usage pharmaceutique.

Selon un troisième aspect, la présente invention a pour objet un procédé de détection, quantification, et tri de molécules d'ADN double brin comprenant au moins une séquence cible telle que précédemment décrite, qui consiste a) à mettre en contact une solution suspectée de contenir lesdites molécules avec un troisième brin d'ADN par exemple un oligonucléotide marqué, de façon à former une triple hélice stable, et b) à détecter le complexe éventuellement formé entre l'ADN double brin et le troisième brin d'ADN.

Ce procédé est utile notamment dans le cadre de l'analyse des génomes en permettant par exemple la détection d'une séquence d'ADN particulière dans un génome ou le tri de séquences spécifiques.

Le troisième brin d'ADN ou l'oligonucléotide, selon cet aspect de la présente invention, peut être marqué en incorporant un marqueur détectable par des moyens spectroscopiques, photochimiques, biochimiques, immunochimiques ou encore chimiques.

Par exemple, de tels marqueurs peuvent consister en des isotopes radioactifs (32P, 33P, 3H, 35S) ou encore des molécules fluorescentes (5-bromodeoxyuridine, fluorescéine , acétylaminofluorène, digoxigénine).

Le marquage est réalisé de préférence par incorporation de molécules marquées au sein des polynucléotides par extension d'amorces, ou bien par rajout sur les extrémités 5' ou 3'.

Des exemples de marquages non radioactifs sont décrits notamment dans le brevet français n° FR 78 109 75 ou encore dans les articles de Urdea et al. (1988, Nucleic Acids Research, 11: 4937-4957) ou Sanchez-pescador et al. (1988; J. Clin. Microbiol., 26(10):1934-1938).

Selon cet aspect particulier de la présente invention, le troisième brin d'ADN ou l'oligonucléotide peut également être immobilisé sur un support tel que précédemment décrit.

Un quatrième aspect de la présente invention concerne un nécessaire ou un kit pour la purification et/ou la détection de la présence d'un ADN double brin selon l'invention dans un mélange complexe ledit nécessaire comprenant un ou plusieurs oligonucléotides tels que décrits précédemment. Ceux-ci peuvent être immobilisés sur un support, et/ou comprendre un marqueur détectable.

Selon cet aspect de la présente invention, le kit de détection décrit ci-dessus, un tel kit comprendra une pluralité d'oligonucléotides conformes à l'invention qui pourront être utilisées pour détecter des séquences cibles d'ADN double brin d'intérêt.

Ainsi, les oligonucléotides immobilisés sur un support peuvent être ordonnés en matrices telles que les "puces à ADN". De telles matrices ordonnées ont été en particulier décrites dans le brevet US N° 5,143,854, dans les demandes PCT N° WO 90/150 70 et 92/10092.

Des matrices supports sur lesquelles les oligonucléotides ont été immobilisés à une haute densité sont par exemple décrites dans les brevets US N°5,412,087 et dans la demande PCT N°WO 95/11995.

La présente demande sera décrite plus en détail à l'aide des exemples qui suivent, qui doivent être considérés comme illustratifs et non limitatifs.

### Légende des Figures :

Figure 1: Représentation schématique du plasmide pXL3179;
Figure 2: Représentation schématique du plasmide pXL 3296;
Figure 3: Représentation schématique du plasmide pXL3426;
Figure 4: Représentation schématique du plasmide pXL3402;
Figure 5: Représentation schématique du plasmide pXL3678;
Figure 6: Représentation schématique du plasmide pXL3207;
Figure 7: Représentation schématique du plasmide pXL3388;
Figure 8: Représentation schématique du plasmide pXL3579.
Figure 9: Représentations schématiques des plasmides pXL3601 et pXL3977.

### Techniques générales de clonage et de biologie moléculaire.

Les méthodes classiques de biologie moléculaire telles que les digestions par des enzymes de restriction, l'électrophorèse sur gel, la ligation de fragments d'ADN, la transformation dans *E*. *coli,* la précipitation des acides nucléiques, le séquençage etc., sont décrites dans la littérature (Maniatis et al., (1989) Molecular cloning : a laboratory manual, second edition, Cold Spring Harbor Laboratory, Cold Spring Harbor Laboratory Press, New York ; Ausubel et al., (1987) Current protocols in molecular biology, John Willey and Sons, New York). Les enzymes de restriction ont été fournies par New-England Biolabs, Beverly, MA (Biolabs).

Les oligonucléotides sont synthétisés en utilisant la chimie des phosphoramidites protégés en α par un groupement cyanoéthyl (Sinha et al. Nucleic Acids Research, 12 (1984) 4539 ; Giles (1985) avec le synthétiseur automatique d'ADN de la Société Applied Biosystem 394 en utilisant les recommandations du fabricant.

Les oligonucléotides utilisés pour la synthèse de gels d'affinité sont obtenus auprès de la Société Amersham Pharmacia Biotech (Uppsala, Suède) ou de Eurogentec (Seraing, Belgique) et sont utilisés tel quels.

Les souches permettant la réplication des plasmides pCOR, et les conditions de croissance et de sélection des ces plasmides ont déjà été décrits (Soubrier et al., Gene Therapy 6 (1999) 1482).

### Exemple 1: Construction des plasmides

### 1.1 Le plasmide pXL3179 (pCOR -FGF1)

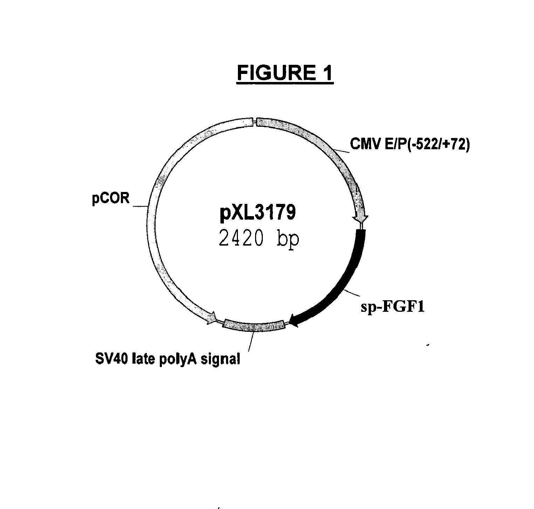

Le plasmide pXL3179 qui est représenté à la Figure 1, est un vecteur dérivé du plasmide pXL2774 (WO97/10343; Soubrier et al., Gene Therapy 6 (1999) 1482) dans lequel le gène codant pour une fusion entre le peptide signal de l'interféron de fibroblastes humain et l'ADNc du FGF1 (Fibroblast Growth Factor 1) (sp-FGF1, Jouanneau et al., PNAS 88 (1991), 2893) a été introduit sous contrôle du promoteur provenant de la région précoce du Cytomégalovirus humain (hCMV IE E/P) et du signal de polyadénylation de la région tardive du virus SV40 (SV40 late polyA; Genbank SV4CG).

### 1.2 Le plasmide pXL 3296 (pCOR)

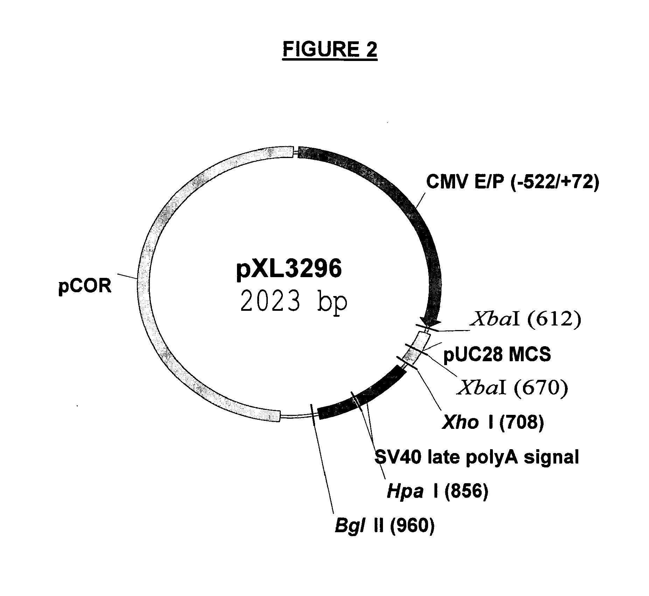

Le plasmide pXL3296 dérive du plasmide pXL3179 dans lequel la séquence du gène sp-FGF1 a été remplacée par le multisite de clonage du plasmide pUC28 (Benes et al., Gene 130 (1993) 151). Le plasmide pXL3296 est représenté à la Figure 2.

### 1.3 Le plasmide pXL3426 (pCOR ― ID1)

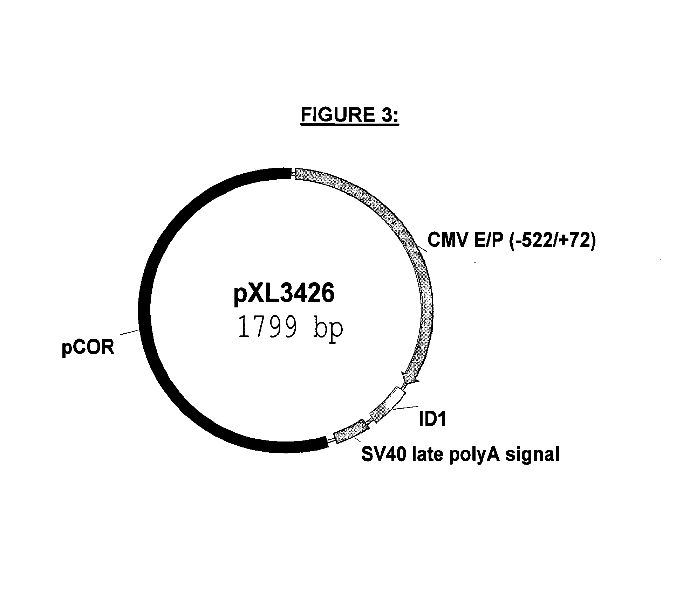

Le plasmide pXL3426 dérive du plasmide pXL3296 dans lequel la séquence 5'-GATCCAAGAAGCATGCAGAGAAGAATTC-3' a été insérée entre les sites *Bg*/II et *Xho*I*.* Le plasmide pXL 3426 est représenté à la Figure 3.

### Exemple 2: Construction d'autres plasmides portant des séquences cibles

Le plasmide pXL3675 dérive du plasmide pXL3296 dans lequel la séquence 5'-GAAGAAGGGAAAGAAGATCTG -3' a été insérée entre les sites Hpal et Xbal; Le plasmide pXL3676 dérive également du plasmide pXL3296 dans lequel la séquence 5'-GAAGAAAGGAGAGAAGATCTG-3' a été insérée entre Hpal et Xbal, et enfin le plasmide pXL3713 qui contient la séquence d'ADN 5'-GAAGAAGTTTAAGAAGATCTG-3' insérée entre les sites Hpal et Xbal du pXL3296. Les plasmides ainsi construits ont été purifiés par gradient en chlorure de CsCl et la séquence des inserts a été confirmée par séquençage. Ces préparations ont été utilisées dans les Exemples décrits ci-après.

### Exemple 3: Identification d'une séquence 20-mer interne au gène FGF1 formant une triple hélice stable

Les différents plasmides tels que décrits dans les exemples qui suivent ont été chromatographiés par chromatographie d'affinité par interaction triple hélice dans des conditions standardisées. Le support d'affinité a été synthétisé de la façon suivante à partir du support chromatographique Sephacryl® S-1000 SF (Amersham Pharmacia Biotech). Dans une première étape, le gel Sephacryl® S-1000 dispersé dans du tampon 0.2 M acétate de sodium a été activé avec du *meta*-periodate de sodium ( 3 mM, 20°C, 1 h), puis dans une seconde étape, l'oligonucléotide a été couplé par l'intermédiaire de sa partie 5'-NH₂ terminale aux groupements aldéhydes de la matrice activée, par une réaction d'amination réductive en présence d'acide ascorbique (5 mM) en suivant une procédure similaire à celle décrite pour le couplage de protéines (Hornsey et al., J. Immunol Methods 93 (1986) 83). Pour toutes les expériences rapportées dans la présente invention, les oligonucléotides ont été couplés en suivant cette procédure générale, tous les oligonucléotides présentent un bras fonctionnalisé NH₂-(CH₂)₆- situé à l'extrémité 5' de l'oligonucléotide.

Les expériences visant à mettre en évidence la formation d'une structure triple hélice entre un oligonucléotide et un ADN double brin et à en mesurer la stabilité, ont toutes été réalisées dans les conditions suivantes. Lors de chaque expérience, 300 µg de plasmide purifié mis en solution dans 6 ml de tampon 50 mM acétate de sodium (pH 4.5) contenant 2 M NaCl a été injecté à un débit de 30 cm/h sur une colonne HR 5/5 (Amersham Pharmacia Biotech) contenant 1 ml de gel d'affinité fonctionnalisé par un oligonucléotide selon l'invention. Après lavage de la colonne avec 5 ml du même tampon, le plasmide a été élué avec 3 ml de colonne tampon 100 mM Tris/HCl (pH 9.0) contenant 0.5 mM EDTA et la quantité de plasmide éluée par le tampon pH 9.0 a été quantifiée i) par mesure de l'absorbance à 260 nm de la solution et ii) par chromatographie d'échange d'anions sur une colonne Millipore GenPak-Fax (Marquet et al., BioPharm, 8 (1995) 26).

A partir d'une colonne fonctionnalisée avec l'oligonucléotide 5'- NH₂-(CH₂)₆ -TT(CTT)₆-3' (SEQ ID N°: 2), les résultats de la purification qui sont présentés dans le Tableau 1 ci-dessous démontrent la formation d'une triple hélice stable avec les plasmides comprenant soit le gène FGF1 entier (pXL3179), soit une séquence interne ID1 du gène humain FGF1 (pXL3426), par opposition à un plasmide contrôle (pXL3296) qui ne comprend aucune séquence du gène humain FGF1 et qui n'est pas retenu sur la colonne en question.

**Tableau 1**

| Plasmides | Séquence de l'oligonucléotide Séquence cible dans le plasmide | Capacité (µg plasmide fixé par ml de gel) |
|---|---|---|
| pXL3179 (pCOR-FGF1) | TT CTT CTT CTT CTT CTT CTT | 175 |
| | - | |
| pXL3426 (pCOR-ID1) | TT CTT CTT CTT CTT CTT CTT | 130 |
| | AA GAA GCA TGC AGA GAA GAA | |
| pXL3296 (pCOR) | TT CTT CTT CTT CTT CTT CTT | <1 |
| | - | |

La séquence du plasmide pXL3426 a été identifiée par sous clonage de différents fragments du gène FGF1 de taille de plus en plus petite. La séquence interne désignée ID1 5' - AA GAA GCA TGC AGA GAA GAA - 3' (SEQ ID N°: 1) du gène FGF1 forme donc une structure triple hélice stable avec l'oligonucléotide utilisé de séquence SEQ ID N°: 2. La structure triple hélice obtenue contient deux zones de type Pyrimidine-Purine-Pyrimidine (Py*PuPy) formant des triades canoniques T*AT et ⁺C*GC longues de 6 unités (R, côté 5') et de 7 unités (R', côté 3') séparées par une zone interne (T) de 7 triades dont six d'entre elles sont non canoniques et comprennent plus précisément deux triades T*GC, deux triades T*CG, une triade C*AT, et une triade C*TA.

### Exemple 4: Identification des bases nécessaires au sein de la séquence 20-mer interne ID1 au gène FGF1 à la stabilité de la structure triple hélice

A partir de la séquence interne ID1, 4 oligonucléotides ont été préparés. Pour deux d'entre eux, 7 ou 13 nucléotides sont absents du côté 5' de ID1, et pour les 2 autres, 7 ou 14 nucléotides sont absents du côté 3'. Le plasmide pXL3426 a été chromatographié sur une colonne d'interaction triple hélice fonctionnalisée à l'aide de l'oligonucléotide 5'-TT(CTT)₆-3' (SEQ ID N°: 2) ou des oligonucléotides FRB36, FRB38, FRB39, ou FRB40. La stabilité de la structure triple hélice formée avec les différentes séquences internes ID1 tronquées a été ensuite testée par la mesure de la quantité de chacun des plasmides retenue sur la colonne.

**Tableau 2**

| Oligonucléotide | Séquence ciblée dans le plasmide pXL3426 | Capacité (µg plasmide fixé par ml de gel) |
|---|---|---|
| (CTT)7 | AA GAA GCA TGC AGA GAA GAA | 130 |
| FRB36 | -- --- --- --- -- A GAA GAA | < 1 |
| FRB38 | AAGAAG-- --- --- --- --- | < 1 |
| FRB39 | AA GAA GCA TGC AG - - - - - - - | 22 |
| FRB40 | -- --- --ATGCAGAGAAGAA | 33 |

Les résultats présentés dans le tableau 2 ci-dessus montrent que l'ensemble de la séquence ID1 du pXL3426 (20-mer) est nécessaire à la formation d'une structure triple hélice stable avec un oligonucléotide de type 5'-TT(CTT)₆-3'. En particulier les deux parties Py*PuPy situées aux extrémités 5' et 3' ainsi que la partie centrale contribuent très largement et de façon coopérative à la stabilité de la structure finale.

### Exemple 5: Influence des triades canoniques et du nombre des triades non canoniques sur la stabilité de la triple hélice

La séquence de l'oligonucléotide 5'-TT(CTT)₆-3' (SEQ ID N°: 2) a été modifiée et la capacité de ces différents oligonucléotides (FRB15, FRB16, et FRB17) à former une triple hélice stable avec la séquence interne ID1 (5' - AA GAA GCA TGC AGA GAA GAA - 3' ; SEQ ID N°: 1) du plasmide pXL3426 a été testée.

**Tableau 3**

| Oligonucléotide | Séquence de l'oligonucléotide Séquence ID1 cible dans le plasmide | Nombre de triades non canoniques | Capacité (µg plasmide fixé par ml de gel) |
|---|---|---|---|
| (CTT)7 | TT CTT CTT CTT CTT CTT CTT | 6 | 130 |
| | AA GAA GCA TGC AGA GAA GAA | | |
| FRB15 | TT CTT CTT GCT TCT CTT CTT | 3 | 189 |
| | AA GAA GCA TGC AGA GAA GAA GAA | | |
| FRB1 | TT CTT CTT GTT TCT CTT CTT | 4 | 171 |
| | AA GAA GCA TGC AGA GAA GAA | | |
| FRB17 | TT CTT CTT CCT TCT CTT CTT | 3 | 183 |
| | AA GAA GCA TGC AGA GAA GAA | | |

Les résultats présentés dans le Tableau 3 ci-dessus montrent qu'il est possible d'accroître la stabilité de la structure triple hélice en modifiant la séquence de l'oligonucléotide de façon à augmenter le nombre de triades canoniques T*AT et ⁺C*GC, ce qui dans le même temps réduit le nombre des triades non canoniques dans la zone interne médiane N de la structure triple hélice.

### Exemple 6: Influence des triades non canoniques sur la stabilité de la structure de la triple hélice

La séquence du plasmide pXL3426 comprenant la séquence interne ID1 (SEQ ID N°: 1) du gène FGF1 qui est susceptible de former une triple hélice stable avec l'oligonucléotide 5'-TT(CTT)₆-3' (SEQ ID N°: 2), a été modifiée afin d'introduire dans la zone centrale N deux triades non canoniques identiques consécutives du type T*GC suivies en 5' d'une triade non canonique C*AT (pXL3675). Dans une autre expérience, ont été introduites cinq triades non canoniques successives C*AT, T*GC, T*GC, C*AT, et T*GC (pXL3676). Enfin, la séquence du plasmide pXL3426 a été modifiée de façon à introduire dans la zone médiane deux triades non canoniques consécutives du type T*TA suivies en 5' d'une triade non canonique C*TA (pXL3713).

**Tableau 4**

| Plasmides | Séquence de l'oligonucléotide Séquence cible dans le plasmide | Capacité (µg plasmide fixé par ml de gel) |
|---|---|---|
| pXL3426 | TT CTT CTT CTT CTT CTT CTT | 112 |
| | AA GAA GCA TGC AGA GAA GAA | |
| pXL3675 | CTT CTT CTT CTT CTT CT | 99 |
| | GAA GAA GGG AAA GAA GA | |
| pXL3676 | CTT CTT CTT CTT CTT CT | 78 |
| | GAA GAA AGG AGA GAA GA | |
| pXL3713 | CTT CTT CTT CTT CTT CT | <1 |
| | GAA GAA GTT TAA GAA GA | |

Les résultats de purification des différents plasmides qui sont présentés dans le Tableau 4 ci-dessus montrent qu'une structure triple hélice stable est formée lorsque la zone centrale non canonique forme des triades de type T*CG, T*GC, C*AT et C*TA. Comme le montre le rendement de fixation sur gel d'affinité des plasmides pXL3675 et pXL3676, une triple hélice stable est également formée lorsque deux triades non canoniques T*GC consécutives sont introduites et quel que soit le contexte, c'est à dire que les triades environnantes soient de nature canonique ou non canonique. Par contre, l'introduction d'une paire de triades contiguës de type T*TA et d'une triade C*TA contiguës conduisent à une déstabilisation complète de la structure triple hélice.

### Exemple 7: Constructions de plasmide comprenant une cassette codant pour un gène SeAP, hαFP, FIX et GAX

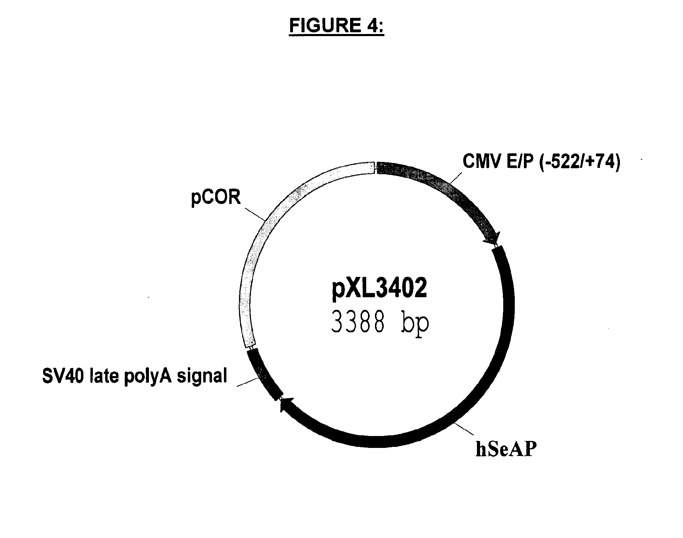

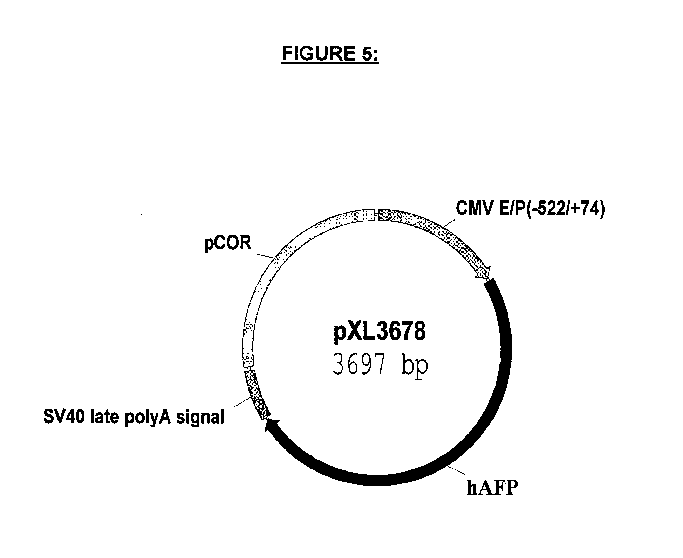

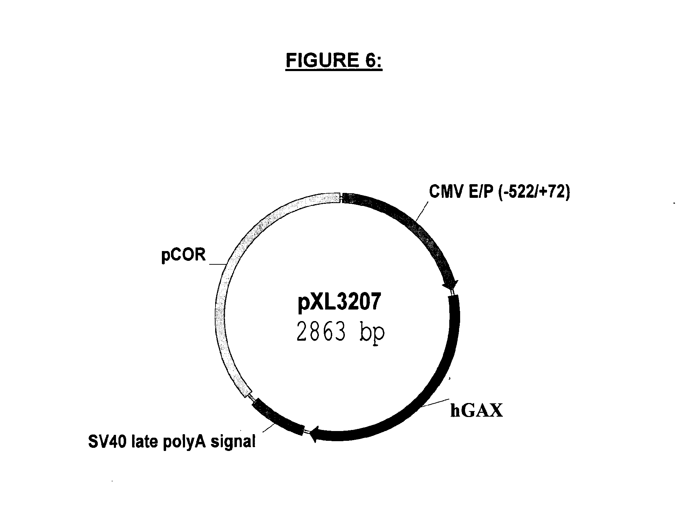

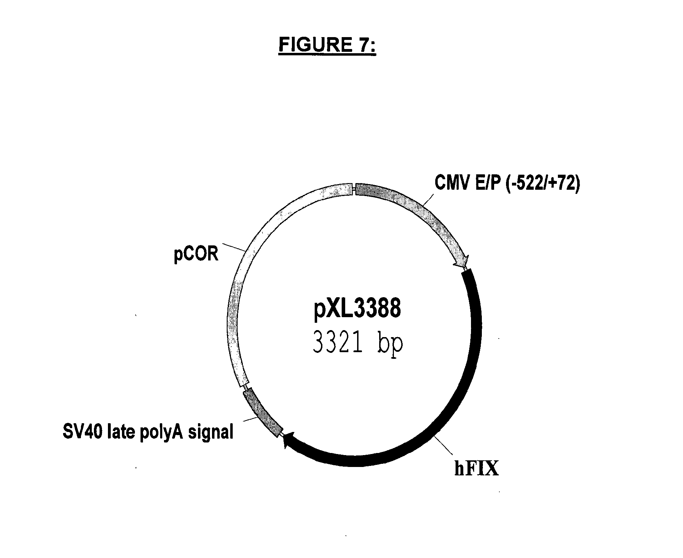

Les gènes utilisés dans ces expériences pour mettre en évidence l'activité des compositions de l'invention sont par exemples le gène humain codant pour le facteur F IX (Kurachi et al., Proc. Natl. Acad. Sci. U.S.A. 79 (1982) 6461), le gène humain codant pour la phosphatase alcaline sécrétée SeAP (Millan et al., J. Biol. Chem., 261 (1986) 3112), le gène humain codant pour l'alpha foeto-protéine hαFP (Gibbs et al., Biochemistry 26 (1987) 1332), le gène humain codant pour GAX (Gorski et al., Mol. Cell. Biol., 13 (1993) 3722). Ces gènes ont été amplifiés par PCR à partir de plasmides ou banques d'ADNc (Clontech) puis clonés en aval du promoteur eucaryote CMV E/P et en amont de la séquence SV40 late polyA signal dans un plasmide pCOR dérivé de pXL3296. Le gène codant pour la phosphatase alcaline sécrétée (SeAP) a été introduit dans un plasmide pCOR dérivé de pXL3296 pour générer le plasmide pXL3402 (Figure 4). Le gène codant pour l'alpha foeto-protéine (hαFP) a été introduit dans un plasmide pCOR dérivé de pXL3296 pour générer le plasmide pXL3678 (Figure 5). Le gène codant pour GAX a été introduit dans un plasmide pCOR dérivé de pXL3296 pour générer le plasmide pXL3207 (Figure 6). Le gène codant pour le facteur FIX a été introduit dans un plasmide pCOR dérivé de pXL3296 pour générer le plasmide pXL3388 (Figure 7).

### Exemple 8: Utilisation d'un oligonucléotide de type 5'-(CTT)7-3 ' pour générer la formation de structures triple hélice stables avec divers gènes d'intérêt

L'interaction de différentes séquences avec le gel d'interaction triple hélice fonctionnalisé par l'oligonucléotide 5'-TT(CTT)₆-3' (SEQ ID N°: 2) a été étudiée par la mesure de la capacité obtenue avec des plasmides portant divers gènes. Les gènes étudiés étaient i) le gène humain codant pour le facteur IX, ii) le gène de la phosphatase alcaline sécrétée SeAP, iii) le gène humain de l'alpha foeto-protéine (αFP) et iv) le gène humain GAX .

**Tableau 5**

| Gènes | Séquence(s) cible(s) dans le gène | Capacité (µg plasmide fixé par ml de gel) |
|---|---|---|
| Facteur IX | GAA GAA GCA CGA GAA G | 71 |
| SEAP | AA GAA AGC AGG GAA G GAA GAG GAA GAA G | 100 |
| hαFP | AAG GAG AAG AAG AA | 146 |
| hGAX | AA GAT GAG GAA GAA G | 118 |

Les résultats présentés dans le Tableau 5 ci-dessus montrent qu'il est possible en utilisant un oligonucléotide de type (CTT)ₙ de former des structures triple hélice stables avec un gène d'intérêt bien que ce gène ne contienne pas de séquence cible de type 5'-(GAA)ₙ-3' complémentaire de l'oligonucléotide. L'existence dans la partie centrale de la séquence cible de bases engagées dans des triades de type T*CG, C*CG, T*GC, et C*AT, est tolérée par la structure triple hélice, de même que la présence d'une triade isolée T*TA (gène GAX).

### Exemple 9: Utilisation d'une colonne fonctionnalisée par un oligonucléotide de type 5'-(CTT)₇-3 pour la purification d'un plasmide contenant la séquence interne ID1 (5' -AA GAA GCA TGC AGA GAA GAA - 3' : SEQ ID N°: 1)

La possibilité de purifier des plasmides portant une séquence de type 5'-(R)ₙ-(N)ₜ-(R')ₘ-3', telle que décrite précédemment, à l'aide d'un support chromatographique d'affinité par interaction triple hélice fonctionnalisé avec un oligonucléotide de type 5'-(CTT)₇-3' a été étudiée a partir de l'Exemple 8.

Le plasmide pXL3179 (comportant le gène FGF1 humain, porteur de la séquence 5' - AA GAA GCA TGC AGA GAA GAA - 3' a été chromatographié sur une colonne d'interaction de Sephacryl S-1000 fonctionnalisée avec l'oligonucléotide 5'-NH₂-(CH2)₆-(CTT)₇-3'. Pour cela, 9.40 mg de plasmide pXL3179 dans 60 ml de tampon 50 mM acétate de sodium, 2 M NaCl (pH 4.5) ont été injectés à un débit de 30 cm/h sur une colonne d'affinité de 10 ml contenant l'oligonucléotide 5'-NH₂-(CH2)₆-(CTT)₇-3' couplé de façon covalente à du Sephacryl S-100 SF comme décrit dans l'Exemple 3. Après lavage de la colonne avec 5 volumes du même tampon, le plasmide fixé a été élué avec 2 volumes de colonne de tampon 100 mM Tris/HCl, 0.5 mM EDTA et quantifié par mesure de l'absorbance UV (260 nm) et par chromatographie d'échange d'ions sur une colonne GenPak-Fax (Waters). La teneur en ADN génomique de E. *coli* dans la préparation initiale et dans la fraction purifiée a été mesurée par PCR comme décrit dans W0 96/18744. 7.94 mg de plasmide pXL3179 a été retrouvé dans la fraction éluée (rendement d 'élution, 84 %) et le niveau de contamination par l'ADN génomique de E.Coli a été réduit de 7.8 à 0.2 % par la chromatographie d'affinité décrite. De même, le niveau de contamination par les ARN a été réduit de 43 % dans le plasmide de départ à 0.2 % dans le plasmide purifié.

Dans diverses autres expériences de chromatographie effectuées à partir de diverses préparations de plasmide pXL3179 chromatographiées sur une colonne d'affinité de Sephacryl S-1000 fonctionnalisée par l'oligonucléotide 5'-NH₂-(CH2)₆-(CTT)₇-3', la teneur en ADN génomique a été réduite de 0.2 % à 0.007 %, de 0.7 % à 0.01 %, de 7.1 % à 0.2 %, ou de 7.8 %à0.1 %.

### Exemple 10: Utilisation d'un oligonucléotide de type 5'-CCT TTT CCT CCT T-3 ' (SEQ ID N :12) pour générer la formation de structures triple hélice stables avec un gène d'intérêt thérapeutique VEGFB-167 humain

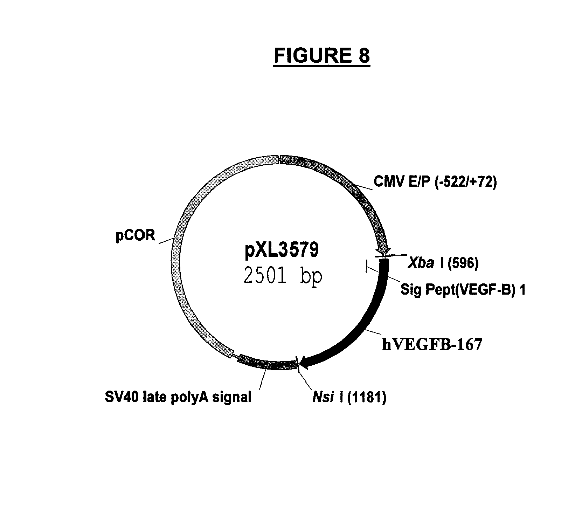

L'interaction d'une séquence interne à un gène d'intérêt thérapeutique tel que le gène VEGFB-167 humain, avec un support d'interaction triple hélice fonctionnalisé par l'oligonucléotide 5'-CCT TTT CCT CCT T-3 (SEQ ID N°: 12) a été étudiée par la mesure de la capacité obtenue avec le plasmide pXL3579 portant le gène VEGFB-167 humain (Figure 8) (Olofsson et al., J. Biol. Chem., 271 (1996) 19310). Le plasmide pXL3579 qui est représenté à la Figure 8, contient le gène VEGF-B167 amplifié par PCR à partir d'une banque d'ADNc de coeur humain (Clontech) puis cloné en aval du promoteur eucaryote CMV E/P (-522/+74) et en amont de la séquence SV40 late polyA signal entre les sites Nsil et Xbal du multisite de clonage de pXL3296.

**Tableau 6**

| Oligonucléotide | Séquence cible dans le gène (VEGFB-167 ou contrôle) | Capacité (µg plasmide fixé par ml de gel) |
|---|---|---|
| CCT TTT CCT CCT T | GGC AAC GGA GGA A | 87 |
| | aucune (vecteur contrôle) | < 0.5 |
| CCT CCT T | GGA GGA A | < 0.5 |
| | aucune (vecteur contrôle) | < 0.5 |
| TTT TTT TTC CT | AAA AAA AAG GA | 15 |
| | aucune (vecteur contrôle) | < 0.5 |

Les résultats présentés dans le Tableau 6 ci-dessus montrent qu'il est possible en utilisant un oligonucléotide, tel que par exemple l'oligonucléotide 5' - CCT TTT CCT CCT T - 3' (SEQ ID N°12), ciblant une séquence de type 5'-(R)ₙ-(N)ₜ-(R')ₘ-3', ici la région 5' - GGC AAC GGA GGA A - 3' (SEQ ID N° :13 ) du gène VEGFB-167 humain, de former une structure triple hélice stable avec une région d'un gène d'intérêt. Bien que par ailleurs, le gène VEGFB-167 humain contienne une séquence homopurique 5' - AAA AAA AAG GA - 3' ciblée par l'oligonucléotide 5' - TTT TTT TTC CT - 3' (Tableau 6), l'interaction obtenue avec l'oligonucléotide 5' - CCT TTT CCT CCT T - 3' est très largement supérieure à l'interaction obtenue avec l'oligonucléotide homopyrimidique. De même, la séquence interne homopurique 5' - GGA GGA A - 3' n'est pas suffisamment longue pour permettre la formation d'une triple hélice stable avec l'oligonucléotide 5'- CCT CCT T ― 3'.

Cet exemple montre donc clairement l'intérêt des séquences de type 5'-(R)ₙ-(N)ₜ-(R')ₘ-3' pour la formation des structures triple hélice stables, même dans un contexte où l' ADN double brin étudié présente par ailleurs au moins une structure homopurique de longueur importante.

### Exemple 11: Utilisation d'un oligonucléotide de type 5'-T CCT CTCCCT C-3 ' (SEQ ID N .14) pour la séparation de l'ADNc du VEGFB-186 non délété via la formation de structures triple hélice stables avec une séquence cible au sein de l'ADNc du VEGFB-186 modifié

Il a été observé, au cours des étapes de production en fermenteur du gène VEGFB-186 humain, que celui-ci était le siège de réarrangements et délétions génétiques notamment au niveau de l'exon 6A. Des mutations ponctuelles silencieuses ont donc été introduites par PCR séquentielle et mutagénéisante au niveau des nucléotides 510 (A/C), 513 (C/T), 516 (A/T), 519 (C/T), et 522 (C/T) par rapport au point + 1 de traduction. La séquence en acides aminés de la protéine VEGFB-186 comprise entre l'acide aminé 170 et 174 reste inchangée. Par contre, le gène VEGFB-186 ainsi modifié (VEGFB-186m) présente une séquence d'ADN cible selon la présente invention 5'-A GGA GCG GGA G-3' (SEQ ID NO: 15), qui est capable de former une interaction triple hélice stable avec un oligonucléotide de séquence 5'-T CCT CTC CCT C-3' (SEQ ID NO: 14). Cette interaction tripe hélice stable est avantageusement utilisée pour mettre en oeuvre le procédé selon l'invention, et séparer le gène VEGFB-186 modifié n'ayant pas subi de réarrangements et délétions après sa production en fermenteur.

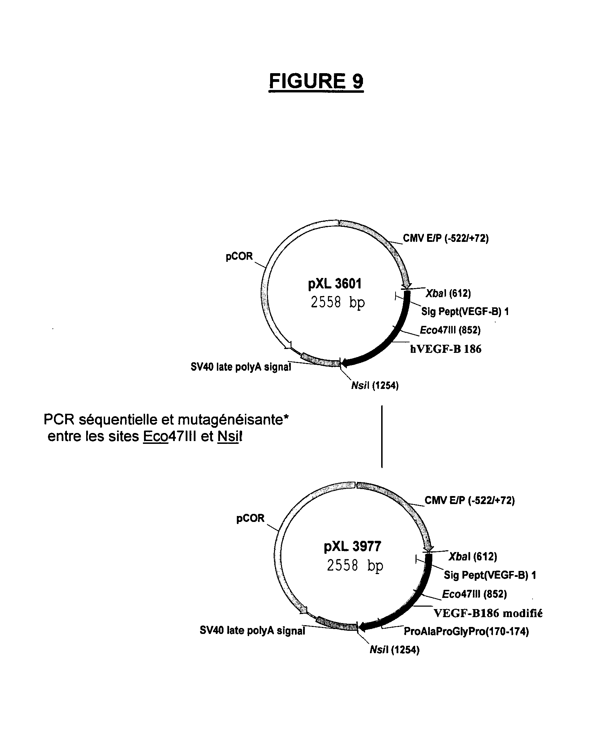

Afin d'illustrer le procédé de purification par interaction triple hélice du gène VEGFB-186 modifié, deux plasmides pXL3601 et pXL3977, tels que représentés à la Figure 9, ont été utilisés. Le gène VEGF-B186 a été tout d'abord amplifié par PCR à partir d'une banque d'ADNc de coeur humain (Clontech), puis cloné en aval du promoteur eucaryote CMV E/P (-522/+74) et en amont du signal de polyadénylation de la région tardive du virus SV40 entre les sites Nsil et Xbal du multisite de clonage de pXL3296 (Exemple 1.2), afin de générer le plasmide pXL3601. Ce dernier a été modifié par PCR séquentielle et mutagénéisante, afin de générer le plasmide pXL3977 dans lequel le gène VEGFB-186 est modifié au niveau de l'exon 6A comme décrit ci-dessus.

L'interaction de la séquence cible 5'-A GGA GCG GGA G-3' (SEQ ID NO: 15) au sein du gène VEGFB-186m, avec un support d'interaction triple hélice fonctionnalisé par l'oligonucléotide 5'-T CCT CTC CCT C-3' (SEQ ID N°: 14) a été étudiée par la mesure de la capacité obtenue avec le plasmide pXL3977 portant le gène VEGFB-186 humain modifié (Figure 9). La séquence du VEGFB-167 étant contenue dans la séquence du VEGFB-186m , le plasmide pXL3579 tel que décrit dans l'exemple 10 et qui comprend le gène VEGFB-167 humain est donc utilisé comme contrôle négatif.

**Tableau 7**

| Oligonucleotide | pCOR-VEGF-B186m (pXL3977) (µg plasmide fixé par ml de gel) | PCOR-VEGF-B167 (pXL3579) (µg plasmide fixé par ml de gel) | pCOR vide (pXL3296) (µg plasmide fixé par ml de gel) |
|---|---|---|---|
| ID n°14 | 234 | 32 | < 1.0 |
| ID n°16 | 224 | 0.5 - 1.0 | < 1.0 |

Les résultats présentés dans le Tableau 7 ci-dessus montrent qu'il est possible en utilisant un oligonucléotide, tel que par exemple l'oligonucléotide (SEQ ID N°14), ciblant une séquence de type 5'-(R)ₙ-(N)ₜ-(R')ₘ-3', ici la région 5' ― A GGA GCG GGA G- 3' (SEQ ID N° :15 ) du gène VEGFB-186 humain modifié, de former une structure triple hélice stable avec une région d'un gène d'intérêt, et de ainsi le purifier de manière efficace. L'oligonucléotide 5'-TTT CCT CTC CCT C-3' (SEQ ID N°16) peut être également utilisé pour la purification du gène VEGFB-186 humain modifié.

## Revendications

1. Procédé de purification d'une molécule d'ADN double brin **caractérisé en ce que** l'on met en contact ladite molécule d'ADN double brin avec un troisième brin d'ADN, ladite molécule d'ADN double brin comprenant au moins une séquence cible de formule générale:
5'-(R)ₙ-(N)ₜ-(R')ₘ-3'
dans laquelle :
R' et R' représentent des séquences nucléotidiques composées uniquement de bases puriques ;
n et m sont des nombres entiers inférieurs à 9 ; et la somme n+m est supérieure à 5 ;
N est une séquence nucléotidique comprenant à la fois des bases puriques et des bases pyrimidiques ; et
t est un nombre entier inférieur à 8 ;
ladite séquence d'ADN étant susceptible d'interagir avec un troisième brin d'ADN pour former une structure triple hélice.

2. Procédé selon la revendication 1, **caractérisé en ce que** l'interaction entre la région N et le troisième brin d'ADN conduit à la formation d'au plus 6 triades non canoniques.

3. Procédé selon la revendication 2, **caractérisé en ce que** les triades non canoniques ainsi formées sont choisies parmi les triades T*CG, T*GC, C*AT, et C*TA.

4. Procédé selon l'une quelconque des revendications précédentes, **caractérisé en ce que** les régions R et R' comprennent au moins deux guanines (G) au total.

5. Procédé selon l'une quelconque des revendications précédentes, **caractérisé en ce que** les régions R et R' comprennent au moins 2 adénines.

6. Procédé selon l'une quelconque des revendications précédentes, **caractérisé en ce que** les régions R et R' forment avec le troisième brin d'ADN des triades canoniques choisies parmi T*AT et ⁺C*GC.

7. Procédé selon l'une quelconque des revendications précédentes, **caractérisé en ce que** le troisième brin d'ADN est de type homopyrimidique.

8. Procédé selon la revendication 7, **caractérisé en ce que** le troisième brin d'ADN comprend une séquence poly-CTT.

9. Procédé selon l'une quelconque des revendications précédentes, **caractérisé en ce que** troisième brin d'ADN est un oligonucléotide.

10. Procédé selon l'une quelconque des revendications précédentes, **caractérisé en ce que** la séquence cible présente sur ladite molécule d'ADN double brin comprend la séquence SEQ ID N°:1, et ledit oligonucléotide est choisi parmi les oligonucléotides de séquence SEQ ID N°: 2 à 5.

11. Procédé selon l'une des revendications précédentes, **caractérisé en ce que** la séquence cible est naturellement présente sur la molécule d'ADN double brin, ou est une séquence cible introduite de manière artificielle au sein de la molécule d'ADN double brin.

12. Procédé selon l'une des revendications précédentes **caractérisé en ce que** la séquence cible naturellement présente sur la molécule d'ADN est présente dans la séquence codante de gènes d'intérêt thérapeutique ou expérimental.

13. Procédé selon la revendication 12, **caractérisé en ce que** la séquence d'ADN cible comprend tout ou partie de la séquence SEQ ID N°:1 comprise dans le gène humain FGF1.

14. Procédé selon la revendication 12, **caractérisé en ce que** la séquence d'ADN cible comprend la séquence SEQ ID N°:6 comprise dans le gène humain codant pour le facteur IX.

15. Procédé selon la revendication 12, **caractérisé en ce que** la séquence d'ADN cible comprend la séquence SEQ ID N°:7 ou N°:8 comprise dans le gène humain de la phosphatase alcaline sécrétée.

16. Procédé selon la revendication 12, **caractérisé en ce que** la séquence d'ADN cible comprend la séquence SEQ ID N°:9 comprise dans le gène humain de l'alpha foeto-protéine (αFP).

17. Procédé selon la revendication 12, **caractérisé en ce que** la séquence d'ADN cible comprend la séquence SEQ ID N°:10 comprise dans le gène humain GAX.

18. Procédé selon la revendication 12, **caractérisé en ce que** la séquence d'ADN cible comprend la séquence SEQ ID N°:13 comprise dans le gène humain VEGFB167.

19. Procédé selon l'une quelconque des revendications précédentes, **caractérisé en ce que** la molécule d'ADN double brin est un ADN circulaire, tel qu'un plasmide, un minicercle, un fragment linéaire.

20. Procédé de purification selon l'une quelconque des revendications précédentes, **caractérisé en ce que** l'oligonucléotide est couplé de façon stable, covalente ou non covalente sur un support.

21. Procédé selon l'une quelconque des revendications précédentes, **caractérisé en ce que** l'oligonucléotide est greffé à un polymère d'origine naturelle ou synthétique.

22. Procédé selon la revendication 20 **caractérisé en ce que** le support est choisi parmi les supports de chromatographie, les surfaces plastiques et les billes de latex fonctionnalisés.

23. Procédé selon la revendication 22, **caractérisé en ce que** le support de chromatographie est un support pour la perméation de gel.

24. Procédé selon l'une quelconque des revendications précédentes, **caractérisé en ce que** la solution contenant ladite molécule d'ADN double brin est un lysat cellulaire.

25. Procédé selon l'une quelconque des revendications précédentes, **caractérisé en ce que** le lysat cellulaire est un lysat clair.

26. Procédé selon l'une des revendications précédentes, **caractérisé en ce que** la molécule d'ADN double brin est pré-purifiée.

27. Procédé selon l'une des revendications 20 à 26, **caractérisé en ce que** l'oligonucléotide possède la séquence GAGGCTTCTTCTTCTTCTTC TTCTT (SEQ ID N°:11).

28. Procédé selon l'une des revendications 20 à 27, **caractérisé en ce que** l'oligonucléotide est couplé au support par liaison disulfure, thioéther, ester, amide ou amine.

29. Procédé selon l'une des revendications 20 à 28, **caractérisé en ce que** l'oligonucléotide est fixé au support par l'intermédiaire d'un bras composé d'une chaîne carbonée (CH₂)ₙ dans laquelle n est un nombre entier compris entre 1 et 18 inclus, ledit bras étant relié à l'oligonucléotide par un phosphate et au support par une liaison amine.

30. Procédé selon l'une des revendications précédentes, **caractérisé en ce que** l'oligonucléotide présente au moins une modification chimique le rendant résistant aux, ou protégé vis-à-vis des nucléases, ou augmentant son affinité pour la séquence spécifique.

31. Procédé selon l'une quelconque des revendications précédentes, **caractérisé en ce que** l'oligonucléotide comprend une séquence dans laquelle une au moins des cytosines est méthylée en position 5'.

32. Procédé pour la purification selon l'une quelconque des revendications précédentes, **caractérisé en ce qu'**il comprend au moins une étape où l'on met en contact une solution contenant ledit ADN double brin, en mélange avec d'autres composants, avec le support auquel est couplé de manière covalente ledit oligonucléotide.

33. Procédé pour la purification selon l'une quelconque des revendications précédentes, **caractérisé en ce que** l'on fait passer une solution contenant ledit ADN double brin, en mélange avec d'autres composants, sur le support de chromatographie auquel est couplé de manière covalente ledit oligonucléotide.

34. Procédé selon l'une quelconque des revendications précédentes, **caractérisé par le fait que** ledit troisième brin d'ADN ou ledit oligonucléotide est marqué.

35. ADN double brin purifié susceptible d'être obtenu par le procédé selon l'une quelconque des revendications précédentes.

36. Procédé de détection d'un ADN double brin **caractérisé en ce que** l'on met en contact une solution suspectée de contenir ladite molécule d'ADN double brin avec un troisième brin d'ADN marqué capable de former, par hybridation une triple hélice avec une séquence cible dudit ADN double brin, ladite séquence cible ayant la formule générale suivante:
5'-(R)ₙ-(N)ₜ-(R)ₘ-3'
dans laquelle :
R' et R' représentent des séquences nucléotidiques composées uniquement de bases puriques ;
n et m sont des nombres entiers inférieurs à 9 ; et la somme n+m est supérieure à 5 ;
N est une séquence nucléotidique comprenant à la fois des bases puriques et des bases pyrimidiques ; et
t est un nombre entier inférieur à 8.


SEQUENCE LISTING

<110> AVENTIS PHARMA SA

<120> PROCEDES DE PURIFICATION ET DE DETECTION DE SEQUENCES CIBLES
D'ADN PAR INTERACTION TRIPLE HELICE

<130> ST01011

<140> ST01011
<141> 2001-03-21

<160> 20

<170> PatentIn version 3.0

<210> 1
<211> 20
<212> DNA
<213> Homo sapiens

<400> 1
aagaagcatg cagagaagaa 20

<210> 2
<211> 20
<212> DNA
<213> Artificial

<220>
<223> Oligonucleotide

<400> 2
ttcttcttct tcttcttctt 20

<210> 3
<211> 20
<212> DNA
<213> Artificial

<220>
<223> Oligonucleotide

<400> 3
ttcttcttgc ttctcttctt 20

<210> 4
<211> 20
<212> DNA
<213> Artificial

<220>

<223> Oligonucleotide

<400> 4
ttcttcttgt ttctcttctt 20

<210> 5
<211> 20
<212> DNA
<213> Artificial

<220>

<223>  Oligonucleotide

<400>  5
ttcttcttcc ttctcttctt                                                    20


<210>  6
<211>  16
<212>  DNA
<213>  Homo sapiens


<400>  6
gaagaagcac gagaag                                                        16


<210>  7
<211>  16
<212>  DNA
<213>  Homo sapiens



<400>  7
aaagaaagca gggaag                                                        16


<210>  8
<211>  13
<212>  DNA
<213>  Homo sapiens



<400>  8
gaagaggaag aag                                                           13


<210>  9
<211>  14
<212>  DNA
<213>  Homo sapiens



<400>  9
aaggagaaga agaa                                                          14


<210>  10
<211>  15
<212>  DNA
<213>  Homo sapiens



<400>  10
aagatgagga agaag                                                         15


<210>  11
<211>  25
<212>  DNA
<213>  Artificial


<220>

<223>  Oligonucleotide

<400>  11
gaggcttctt cttcttcttc ttctt                                              25

<210>  12
<211>  13
<212>  DNA
<213>  Artificial

<220>

<223>  Oligonucleotide

<400>  12
ccttttcctc ctt                                                          13

<210>  13
<211>  13
<212>  DNA
<213>  Homo sapiens

<400>  13
ggcaacggag gaa                                                          13

<210>  14
<211>  11
<212>  DNA
<213>  Artificial

<220>

<223>  Oligonucleotide

<400>  14
tcctctccct c                                                            11

<210>  15
<211>  11
<212>  DNA
<213>  Homo sapiens

<400>  15
aggagcggga g                                                            11

<210>  16
<211>  13
<212>  DNA
<213>  Artificial

<220>

<223>  Oligonucleotide

<400>  16
tttcctctcc ctc                                                          13

<210>  17
<211>  28
<212>  DNA
<213>  Artificial

<220>

<223>  Oligonucleotide

<400>  17
gatccaagaa gcatgcagag aagaattc                                          28

<210>  18
<211>  21
<212>  DNA
<213>  Artificial

<220>

<223>  Oligonucleotide

<400>  18
gaagaaggga aagaagatct g                                                21

<210>  19
<211>  21
<212>  DNA
<213>  Artificial

<220>

<223>  Oligonucleotide

<400>  19
gaagaaagga gagaagatct g                                                21

<210>  20
<211>  21
<212>  DNA
<213>  Artificial

<220>

<223>  Oligonucleotide

<400>  20
gaagaagttt aagaagatct g                                                21